# EUROPEAN PATENT SPECIFICATION

(11) **EP 1 064 855 B1**
(45) Date of publication and mention of the grant of the patent: **02.08.2017**
(21) Application number: 99909229.9
(22) Date of filing: 18.03.1999
(51) Int. Cl.: A61Q 19/00, A61K 36/31, A61P 3/06, A23L 33/105, A23L 19/00

(54) **FOODS RELATING TO LOWERING HUMAN CHOLESTEROL LEVEL**
LEBENSMITTEL ZUR SENKUNG DES MENSCHLICHEN CHOLESTERINSPIEGELS
ALIMENTS VISANT A REDUIRE LE TAUX DE CHOLESTEROL CHEZ L'HOMME

(30) Priority: 19.03.1998 JP 7051098; 19.03.1998 JP 7066498; 04.12.1998 JP 34600198
(43) Date of publication of application: 03.01.2001
(73) Proprietor: SUNSTAR INC., Takatsuki-shi, Osaka 569-1195 (JP)
(72) Inventor: SUIDO, Hirohisa, Kawachinagano-shi, Osaka 586-0015 (JP); IKEDA, Ako, Takatsuki-shi, Osaka 569-0811 (JP); TABEI, Toshio, Takatsuki-shi, Osaka 569-0857 (JP); KOTANI, Mayumi, Kobe-shi, Hyogo 653-0024 (JP); FUJITA, Akihito, Takatsuki-shi, Osaka 569-1121 (JP); TAKEUCHI, Akira, Takatsuki-shi, Osaka 569-1041 (JP)
(74) Representative: Gill Jennings & Every LLP
(86) International application number: PCT/JP1999/001354
(87) International publication number: WO 1999/047006

(56) References cited:
- EP-A- 0 089 056
- EP-A- 0 462 021
- EP-A- 0 586 933
- EP-A- 0 799 579
- GB-A- 2 028 341
- JP-A- 1 242 509
- JP-A- 3 168 061
- JP-A- 4 049 243
- JP-A- 5 504 471
- JP-A- 6 122 619
- JP-A- 6 237 736
- JP-A- 6 340 532
- JP-A- 7 017 846
- JP-A- 7 017 847
- JP-A- 7 025 763
- JP-A- 7 061 916
- JP-A- 7 285 876
- JP-A- 7 309 713
- JP-A- 8 000 169
- JP-A- 8 092 055
- JP-A- 8 092 056
- JP-A- 8 175 954
- JP-A- 8 208 451
- JP-A- 8 259 453
- JP-A- 8 308 534
- JP-A- 8 319 433
- JP-A- 8 325 130
- JP-A- 9 107 894
- JP-A- 9 175 982
- JP-A- 9 221 410
- JP-A- 9 291 023
- JP-A- 10 072 336
- JP-A- 57 209 208
- JP-A- 58 135 803
- JP-A- 61 239 855
- JP-A- 62 000 026
- JP-A- 63 123 355
- PATENT ABSTRACTS OF JAPAN vol. 014, no. 562 (C-0788), 13 December 1990 (1990-12-13) & JP 02 242667 A (TOKYO TANABE CO LTD), 27 September 1990 (1990-09-27)
- PATENT ABSTRACTS OF JAPAN vol. 009, no. 036 (C-266), 15 February 1985 (1985-02-15) & JP 59 181220 A (NITTO DENKI KOGYO KK), 15 October 1984 (1984-10-15)
- KEITA SHIBATA, "Shigen Shokubutsu Jiten", K.K. HOKURYUUKAN, 20 December 1959, "Nanakusa" ("Bursa-Pastoris", "Suzushiro"), "Sativus" ("Suzushiro"), Ú"Japonicus" ("Sceleratus")¾, "Indigotica", "Cresson" ("Oobata Netsukebana"), "Brassica Juncea", ("Takana"), "JAPANESE-HORSERADISH", "TURNIP" ("AKANA", "SUGUKI"), "NA", ("NIPPO-OLEIFERA", "PERVIRIDIS", "CHINESE CABBEGE"), "CAPITATA", (GEMMIFERA"), "OLERACEA", "GRAVEOLENS", "CRISPUM", "PUMILA" XP002921972
- "Ryouri Shokuzai Daijiten", Edited by SHUFUNO TOMOSHA, K.K. SHUFUNO TOMOSHA, 20 January 1998, "Botrytis", "Cauliflower", "Bursa-Pastoris", "Suzushiro", "Takana", "Brassica Juncea", "Japanese-Horseradish", "Akana", "Neosuguki", "Turnip", "Capitata", "OleracEA", "PERVIRIDIS", "GRAVEOLENS", "CRISPUM", "LETTUCE", "PUMILA", "CHINESE CABBEGE", "GEMMIFERA", "SATIVUS" XP002921973
- "DAIDOKORO KAMPOU", SUPERVISED BY YUKIO NEMOTO, K.K. OGATA SHUPPAN, 20 MARCH 1991, PAGES 52-97, 250-251, XP002921974

## Description

### TECHNICAL FIELD

The present invention relates to foods containing broccoli and cabbage for use in lowering human serum cholesterol levels.

### BACKGROUND ART

Recent studies have revealed that the intestinal flora has various functions such as formation or inactivation of carcinogenic substances or senility accelerating substances, and activation or toxification of drugs, in the intestines. In the intestinal flora, Bifidobacteria are known to have functions such as: protection of the body from enteric infection and food poisoning caused by pathogenic bacteria; suppression of intestinal putrefaction to thereby reduce carcinogenesis-related enzymatic activities and formation of putrefaction products; prevention of constipation by promotion of intestinal motility; improvement of the immuno function of the host by stimulation; decomposition or adsorption of carcinogenic substances; and production of Vitamin B group (J. Antibact. Antifung. Agents Vol.25, No.4, pp 219-231, 1997). Attempts have been made to maintain predominance of Bifidobacteria having these functions in the intestines, by administering to humans *Bifidobacterium* proliferation promoting materials by themselves, or foods or drugs containing such materials.

Known *Bifidobacterium* proliferation promoting materials include saccharides such as N-acetylglucosamine, lactulose, raffinose, stachyose, maltotriose ("Bifidobacteria", p 77, 1979, Yakult Honsha Co., Ltd.), fructooligosaccharide ("Kagaku to Seibutsu (Chemistry and Organism", Vol.21, p. 291, 1983, Gakkai Shuppan Center), galactooligosaccharide (Japanese Examined Patent Publications Nos. 1983-20266 and 1986-46479 and Japanese Unexamined Patent Publication No. 1985-41449), isomaltooligosaccharide (Japan Nutritional Science Society, 1986), theanderose (Japanese Unexamined Patent Publication No. 1991-183454), cyclodextrin (Japanese Unexamined Patent Publication No. 1982-138385), and konjac mannan ("Riken Intestinal Flora Symposium, Intestinal Flora and Nutrition", p. 89, 1983, Gakkai Shuppan Center); carrot extract (comprising pantetheine as the main component; "Bifidobacteria", p. 77, 1979, Yakult Honsha Co., Ltd.); soy milk (Japanese Examined Patent Publication No. 1970-9822); soy milk extract (Japanese Unexamined Patent Publication No. 1984-17906); extract of nonpathogenic *Escherichia coli* culture solution (Japanese Examined Patent Publication No. 1975-13359); an enzymatic decomposition product of barley protein-containing material (Japanese Unexamined Patent Publication No. 1986-282070); calabash gourd powder (Japanese Unexamined Patent Publication No. 1988-291579); alcohol extract of residue after extraction of oil soluble components of soybeans (Japanese Unexamined Patent Publications Nos. 1987-155082 and 1985-66978); syrup extracted from calabash gourd fruit (Japanese Unexamined Patent Publication No. 1990-135088); tea extract (Japanese Unexamined Patent Publication No. 1989-191680); Araliaceae family plant extract (Japanese Unexamined Patent Publication No. 1990-249482); a dry potato product (Japanese Unexamined Patent Publication No. 1994-217733); extract of Coffea L. leaves (Japanese Unexamined Patent Publication No. 1994-125771); Chinese cabbage (Japanese Unexamined Patent Publication No. 1990-242667); and citrus fruit (Japanese Unexamined Patent Publication No. 1990-273155).

Many of the known *Bifidobacterium* proliferation promoting materials show a low effect in practical use, or have a low selectivity to enterobacteria so that they are utilized by harmful bacteria other than Bifidobacteria to thereby produce toxic gases. Further, some of the known materials necessitate complicated manipulation for production, or are expensive. Therefore, the known *Bifidobacterium* proliferation promoting materials are not satisfactory in all respects.

Moreover, in recent years, an increased number of middle to senior aged people and children suffer from lifestyle-induced diseases such as myocardial infarction and arteriosclerosis. One of the main risk factors of myocardial infarction and arteriosclerosis is hypercholesterolemia.

Hypercholesterolemia is usually treated by administration of a medicine, in combination with various types of dietary restrictions. However, the medicine predominantly utilized for hypercholesterolemia treatment, i.e., HMG-CoA reductase inhibitor for inhibiting the enzymatic action in the cholesterol synthesis pathway in the body, produces side effects such as rhabdomyolysis and liver functional impairment, and rebound phenomena. Also known is a medicine utilizing an ion exchange resin for adsorbing, in the intestines, bile acid having a high cholesterol content, and discharging the cholesterol from the body. However, the medicine needs to be taken in a great amount and has unpleasant taste. Moreover, long-term dietary restrictions are undesirable since the patient is forced to endure unpleasant meals.

It would therefore be ideal if the serum cholesterol level can be lowered while maintaining an ordinary diet, without dietary restrictions or administration of medicines having side effects such as rhabdomyolysis and liver functional impairement.

In view of the above status, various studies have been made on functional foods effective for lowering human serum cholesterol and improving lipid metabolism.

EP 0 462 021 A and K. Ebihara et al: "Cholesterol-lowering activity of various dietary fibers and their taurocholate-binding capacity in vitro" Database accession no. 1979:53579; & NIPPON NOGEI KAGAKU KAISHI,vol. 52, no. 9, 1978, pages 401-408 disclose the use of an extract or dietary fibres from Japanese radish as cholesterol-lowering agent.
I. Jahodar et al: "A study on the antihypercholesterolemic and antihyperlipidemic effects of cabbage extracts and their phytochemical evaluation" Database accession no. 1994:1007353 ; & PHARMAZIE,vol. 50, no. 12, 1995, pages 833-834 and T. Kiribuchi et al: "Hypocholesterolemic effect of cabbage sterols(l) on plasma and liver cholesterol levels in rats" Database accession no. 1974:131951 ; & EIYO TO SHOKURYO,vol. 26, no. 8, 1973, pages 491-495 disclose the use of cabbage extracts as cholesterol lowering agents.
Hoagland P D: "Binding of Dietary Anions to Vegetable Fiber" Journal of Agricultural and Food Chemistry, American Chemical Society, US LNKD- DOI:10.1021/ JF00089A030, vol. 37, no. 5, 1 September 1989 (1989-09-01), pages 1343-1347, discloses that chenodeoxycholate and decanoate bind to alcohol-insoluble residue of carrot, cabbage, broccoli and onion. The document further suggests that such binding may be beneficial by lowering blood cholesterol levels.

### BRIEF DESCRIPTION OF DRAWINGS

Fig. 2 is a graph showing the *Bifidobacterium* proliferation promoting effect by combined use of specific vegetables.
Fig. 3 is a graph showing the change in mouse serum IgE level with time.

### DISCLOSURE OF THE INVENTION

An object of the present invention is to provide a *Bifidobacterium* proliferation promoting agent containing a *Bifidobacterium* proliferation promoting material which is free from the above drawbacks, has a high *Bifidobacterium* proliferation activity, is capable of being produced with ease at low cost, and is highly safe from the viewpoint of food hygiene; and a food and food material having a *Bifidobacterium* proliferation promoting action. Another object of the present invention is to provide a method for proliferating a *Bifidobacterium.*

The present inventors carried out extensive research to develop, from various natural products, materials having a high *Bifidobacterium* proliferation promoting activity. As a result, they found that vegetables and fruit such as broccoli, cabbage, spinach, *komatsuna (Brassica rapa* L.), *takana* (*Brassica juncea* Czern. et Coss. var. *integrifolia* Sinsk.), *karashina (Brassica juncea* Czern. et Coss.), cauliflower, kale. The object of the present invention is to provide a food having a human serum cholesterol level lowering action, and a preventive or therapeutic agent for human hypercholesterolemia or arteriosclerosis without side effects such as liver functional impairment, the food and agent being free from the above drawbacks.

The present inventors had tested a mixed vegetable juice sold by their company to evaluate its cholesterol level lowering action, by administration in rats. However, no such action had been demonstrated. Therefore, it had been understood that the vegetable juice had no serum cholesterol level lowering action.

However, in a routine medical checkup and subsequent detailed checkup given in the inventors' company, a large number of staff members were found to have a remarkably lowered serum cholesterol level. Research on these staff members revealed that they had not regularly drunk the vegetable juice before the routine checkup but started to drink, after the routine checkup, about one can per day of the juice sold in the company. Consequently, the vegetable juice was surprisingly found to have a human serum cholesterol level lowering action.

The present inventors carried out intense research concerning the effect of the mixed vegetable juice on human serum cholesterol, in order to find out which ingredient showed the action. Further, they extensively tested other edible plants for the action and found that broccoli, shepherd's purse and other edible plants, in particular, functional components in water- soluble fractions of the edible plants, have an inhibitory activity against human hepatocyte cholesterol synthesis, and a good serum cholesterol level lowering action and a good low-density lipoprotein cholesterol (LDL-Chol) level
lowering action, on humans. Moreover, it was surprisingly found that combined use of cabbage, brussels sprouts, Japanese radish or Japanese radish leaves with the above edible plants having the human hepatocyte cholesterol synthesis inhibitory activity specifically enhance the human hepatocyte cholesterol synthesis inhibitory activity to thereby achieve a higher human serum cholesterol level lowering action and a higher human low-density lipoprotein cholesterol (LDL-Chol) level lowering action. Further, the present inventors found that a medicine containing the above edible plants is usable as a preventive or therapeutic agent for human hypercholesterolemia, or a preventive or therapeutic agent for human arteriosclerosis, both free from side effects such as liver functional impairment. The present invention has been accomplished based on the above findings.

The present invention provides the following foods:
[1] A food in accordance with claims 1 to 9 containing broccoli and cabbage and optionally members selected from the group consisting of cauliflower, kale, shepherd's purse, *suzushiro (Arabis flagellosa* Miq.), hatazao *(Arabis glabra), tagarashi (Cardamine flexuosa),* woad, *oobatanetsukebana (Cardamine scutata), yamagarashi (Barbarea vulgaris* R.Br. var. *stricta* Regel), watercress, *takana (Brassica juncea* Czern. et Coss. var. *integrifolia*
   Sinsk.), *karashina (Brassica juncea* Czern. et Coss.), Japanese horseradish, *yuriwasabi (Wasabia tenuis), hinona (Brassica campestris* var. *akana*), *sugukina* (*Brassica campestris* var. *neosuguki*), turnip, rape, spinach, *komatsuna (Brassica rapa* L.), celery, parsley, lettuce, apple, Chinese cabbage, brussels sprouts, Japanese radish and Japanese radish leaves.
[1-A] food according to Item [1] which has a human serum cholesterol lowering action.

The foods of the present invention will be described below in further detail.

The food of the present invention contains broccoli and cabbage and members selected from the group consisting of cauliflower, kale, shepherd's purse, *suzushiro* (*Arabis flagellosa* Miq.), *hatazao (Arabis glabra), tagarashi* (Cardamine flexuosa), woad, *oobatanetsukebana* (*Cardamine scutata*), *yamagarashi* (*Barbarea vulgaris* R.Br. var. *stricta* Regel), watercress, *takana* (*Brassica juncea* Czern. et Coss. var. *integrifolia* Sinsk.), *karashina (Brassica juncea* Czern. et Coss.), Japanese horseradish, *yuriwasabi (Wasabia tenuis), hinona (Brassica campestris*
var. *akana*), *sugukina (Brassica campestris* var. *neosuguki*), turnip, rape, spinach, *komatsuna (Brassica rapa* L.), celery, parsley, lettuce, apple, Chinese cabbage, brussels sprouts, Japanese radish and Japanese radish leaves. These vegetables or fruit are used in combination, in accordance with claims 1 to 9.

The above vegetables and fruit have a human serum cholesterol lowering action. Therefore, the food of the invention, has a human serum cholesterol lowering action.

According to the invention, edible parts are usually utilized, unless otherwise specified. For example, spinach root, which is usually inedible, is not used in the invention. In the case of apple, apple fruit is utilized.

In the present invention, the vegetables or fruit may be used in a raw state, or as thermally sterilized. Alternatively, the raw or thermally sterilized vegetables or fruit may be dried with hot air or freeze-dried, to prepare a film, plate, block or like solid product. Also, a powder prepared by grinding the solid product is usable.

Further alternatively, the raw or thermally sterilized vegetables or fruit may be crushed or squeezed in order to use the resultant product as a whole, or to use only the vegetable or fruit juice obtained. Also usable are a concentrate of the vegetable or fruit juice, or a powder, granules, tablets or like product prepared by drying and processing the concentrate. Further usable are a juice obtained from the raw or thermally sterilized vegetables or fruit using a suitable filter cloth or the like, and a supernatant fluid obtained by centrifugation of the filtrate. The supernatant fluid may be dried with hot air or freeze-dried to obtain a film, plate block or like solid product. The solid product may be ground to obtain a powder.

Thus, for use in the invention, the above vegetables or fruit may be processed by a conventional process comprising any combination of operations such as washing, sorting, peeling, coring, crushing, squeezing, filtration, separation, concentration, heating, cooling, homogenization and drying.

The food [1-A] may consist solely broccoli and cabbage or may contain other ingredients commonly used for foods.

The amount of the other ingredients to be added is not limited but is, for example, about 0.001 to 99.99 wt.% based on the total amount after addition of the other ingredients.

Examples of usable other ingredients include vegetables or fruits other than those listed above, such as lemon, orange, carrot, Japanese radish leaves, pumpkin, sweet pepper, and sweet potato. They are used in the form of a juice, a concentrate, granules or the like obtained by the same process as for the above vegetables or fruit.

Further, additives commonly used for preparation of foods or food materials may be used as other ingredients. Specific examples of usable additives include apple fiber, corn fiber, alginic acid and like fibers; lactose, starch and like excipients; sucrose, maltose, sorbitol, mannitol, oligosaccharide and like sweeteners; vitamins, calcium and like nutritional supplements; thickeners; condiments; colors; etc.

The other vegetables or fruit and additives can be selected according to the intended use and form of the food, the user's taste and other factors, and used in a suitable combination.

Where necessary, the food can be made into or mixed in a desired food product such as: a powder; granules; tablets; capsules; candies; chewing gum; nougat; chocolate; jelly; biscuits; cake; bread; noodles; a beverage; vegetable juice; spread; lactic fermenting beverage, miso(soybean paste), yogurt or like fermented food: fish cake, boiled fish paste or like sea food paste; ham, sausage, processed milk, cheese or like domestic animal food product; or gravy, dressing, sauce, soy sauce or like seasoning. Among these food products, a beverage, in particular vegetable juice, is preferred as the form of the food of the invention.

The food can be used as a food material for preparing a desired food product such as the foods mentioned above. The food material may be used singly to prepare a food product, or may be combined with other food materials to prepare a food product.

The food may be ingested as a mixture with a prepared food such as dehydrated soup or powdered juice, or as a solution or dispersion in canned juice or the like.

Therefore, the food of the invention can be used as a health food, a functional food, a food for specified health use, a food for sick people, etc.

### [1-B]

When the food of the present invention is used as a food having a human serum cholesterol lowering action, the food contains broccoli and cabbage and optionally further contains members selected from sherperd's purse, suzushiro, hatazao, tagarashi, woad, oobatanetsukebane, yamagarashi, watercress, takana, karashina, Japanese horseradish, yuriwasabi, Chinese cabbage, hinona, sugukina, turnip, rape, brussels sprouts, Japanese radish and Japanese radish leaves in accordance with claims 1 to 9. These edible plants have a hepatocyte cholesterol synthesis inhibitory activity, which is an action mechanism to reduce the' human serum cholesterol level most effectively, and to reduce the level of low-density lipoprotein cholesterol (LDL-Chol), a harmful cholesterol. The food of the invention, which contains these plants, have a high human serum cholesterol level lowering action and a high human LDL-Chol level lowering action. Namely, the food of the invention is effective for improving lipid metabolism. The food [1-B] can be ingested as a health food, a functional food, a food for specified health use, a food for sick people, etc.

The food is limited to human use, and is not intended to use as a food or feed for non-human
animals (dogs, cats, rabbits, mice, rats, etc.).

It is particularly preferable that the food contains broccoli and cabbage and additionally at least one member selected from the group consisting of shepherd's purse, *suzushiro, hatazao, tagarashi,* woad, *oobatanetsukebana, yamagarashi,* watercress, *takana, karashina,* Japanese horseradish, *yuriwasabi,* Chinese cabbage, *hinona, sugukina,* turnip and rape, in accordance with claims 1 to 9.

The total content of these plants (vegetables) in the food varies according to the kind of food to be
prepared and differences among the users (individual differences, racial differences, etc.), and is not limited as long as the human serum cholesterol lowering effect can be achieved. It is suitable that the total content is about 0.01 to 100 wt.%, preferably about 1.0 to 99.9 wt.%.

A total content less than 0.01 wt.% is liable to result in insufficient lipid metabolism improving effect, hence undesirable.

The food may contain other ingredients in addition to the above plants, as long as the human serum cholesterol lowering effect is not hindered.

Usable other ingredients include kale, soybeans, oats, barley and like plants known to have a cholesterol lowering action. Also, other plants commonly used in foods, such as celery, lettuce, spinach, *kromatsuna (Brassica rapa* L.), parsley, apple and lemon, which have been processed in the above manner, can be added.

In particular, the food preferably contains at least one member selected from celery, lettuce, spinach, *komatsuna,* parsley, apple and lemon, in addition to the combination of broccoli and cabbage.

The food may contain, as additional ingredients, additives commonly used in the food industry, such as starches, lactose, cellulose, dextrin, sugar alcohols, mucopolysaccharides and like excipients; sucrose, maltose, sorbitol, mannitol, oligosaccharides and like sweeteners; and vitamins, calcium and like nutritional supplements.

The food can be prepared by adding, to the starting materials for preparing a food, the plants processed in the above manner, or the processed plants diluted with a excipient or part of the starting materials, or a solution or dispersion of the processed plants in ethanol, water or the like, at an appropriate stage of the production. The resulting mixture is then mixed in a conventional manner, followed by an ordinary process for preparing the food. The mixing step may be carried out with heating.

Alternatively, the food may be prepared by adding the plants processed in the above manner or a mixture or solution of the processed plants, to a prepared food product, followed by mixing in a conventional manner.

The food is limited to human use, and is not intended to be used as a food or feed for non-human animals (dogs, cats, rabbits, rats, mice, etc.)

The food is not limited in form, and may be made into any of the forms described. Alternatively, the food may be ingested as a mixture with a prepared food product, such as dehydrated soup or powdered juice, or as a solution or dispersion in canned juice.

The intake of the food can be determined according to the form of the food, the age, weight, sex and health conditions of the user, and purpose of intake, without limitation. It is usually preferable that the adult daily intake of the food is about 5 to 1,000 ml when the food is in a liquid form, or about 0.1 to 100 g when the food is in a solid form. The daily intake is preferably divided into several servings.

The food may be taken at any time of day, i.e., before, after or between meals. Intake on before breakfast, lunch or evening meal, or intake on an empty stomach is particularly effective.

Ingestion of the food lowers the human serum cholesterol level without dietary restrictions or programmed exercises.

The food can be used in combination with a conventional preventive or therapeutic agent for hypercholesterolemia, such as Mevalotin. In the combined use, the amount of the conventional agent can be reduced, since the cholesterol lowering action of the food of the invention will compensate the reduction. Thus, the combined use with the food of the invention is advantageous in a long term prevention or treatment, from the viewpoint of side effects.

The vegetables or fruit to be used as active ingredients of the food of the present
invention, have been used as food throughout ages, and therefore can be ingested or administered safely over a long term, for prophylactic or therapeutic purposes.

### BEST MODE FOR CARRYING OUT THE INVENTION

The following Test Examples and Comparative Test Examples are provided to illustrate the present invention in further detail. In these examples, percentages are all by weight (w/w%) unless otherwise specified.

### Test Example 1 Test on hepatocyte cholesterol synthesis inhibitory activity

### (1) Composition and method of preparation of test sample

Juices obtained from the plant samples (1000 g each) shown in Table 1 were filtered through a gauze filter, and the resulting filtrates were centrifugally filtered at 10000 G. The supernatants obtained by the centrifugal filtration were freeze-dried to obtain the test samples shown in Table 2.

**Table 1**

| Composition of plant sample (%) | | | | | | | | |
|---|---|---|---|---|---|---|---|---|
| Plant sample | T-1 | T-2 | T-3 | T-4 | T-5 | T-6 | T-7 | T-8 |
| Broccoli | 100 | - | - | - | - | - | 75 | 75 |
| Shepherd's purse | - | 100 | - | - | - | - | - | - |
| Woad | - | - | 100 | - | - | - | - | - |
| Chinese cabbage | - | - | - | 100 | - | - | - | - |
| Cabbage | - | - | - | - | 100 | - | 25 | - |
| Japanese Radish leaves | - | - | - | - | - | 100 | - | 25 |
| Total | 100 | 100 | 100 | 100 | 100 | 100 | 100 | 100 |

**Table 2 Test sample**

| Plant sample | T-1 | T-2 | T-3 | T-4 | T-5 | T-6 | T-7 | T-8 |
|---|---|---|---|---|---|---|---|---|
| Test sample | S-1 | S-2 | S-3 | S-4 | S-5 | S-6 | S-7 | S-8 |
| Weight of test sample after freeze-drying (g) | 65 | 42 | 54 | 41 | 54 | 36 | 62 | 58 |

### (2) Test method

The Test Samples S-1 to S-8 shown in Table 2 were used as test materials, and tablets containing 5.6% of pravastatin sodium (tradename: Mevalotin) were used as a positive control material. These materials were ground in a mortar and used.

HepG2 cells (2 X 10⁵ cells) cultured in DMEM containing 10% FCS were inoculated into 6-well plates and incubated for 3 days (substantially confluent). After removing the culture media, the test materials S-1 to S-8 and positive control material, all diluted with DMEM and mixed with ¹⁴C-acetic acid, were placed individually in the 6-well plates, followed by 2-hour incubation. Then, the culture media were removed, and the cells were washed with PBS, dissolved in KOH and saponified. The saponified products were subjected to ether extraction and exsiccated.

The exsiccated extracts were dissolved in acetone-ethanol, and 0.5% digitonin was added to each of the solutions. Thereafter, the radioactivity level of the resulting precipitates was measured using a scintillation counter.

### (3) Test result

Table 3 shows the test results, i.e., concentrations of test materials and positive control material which cause 50% inhibition of cholesterol synthesis in HepG2 cells.

**Table 3**

| Concentration for 50% inhibition of cholesterol synthesis | | | |
|---|---|---|---|
| Test sample | Concentration (%) | Titer ratio relative to positive control material | Corrected titer based on yield (Reference: S-7) |
| S-1 | 0.49 | 1/245 | 1/234 |
| S-2 | 0.37 | 1/185 | 1/273 |
| S-3 | 0.46 | 1/230 | 1/264 |
| S-4 | 0.40 | 1/200 | 1/302 |
| S-5 | 1.09 | 1/545 | 1/626 |
| S-6 | 0.78 | 1/390 | 1/672 |
| S-7 | 0.42 | 1/210 | 1/210 |
| S-8 | 0.40 | 1/200 | 1/213 |
| Positive control material (Mevalotin) | 0.002 | 1/1 | 1/1 |

In Table 3, "titer ratio relative to positive control" indicates a value obtained by dividing 0.002% (the concentration of the positive control (Mevalotin) for 50% inhibition of cholesterol synthesis) by the concentration of each test material for 50% inhibition of cholesterol synthesis. Thus, the value indicates the titer ratio of each test material to an equal weight of the positive control material.

"Corrected titer based on yield (reference: S-7)" in Table 3 is a value obtained by correcting the titer ratio relative to positive control based on the yield of each test material found from Table 2, using Test Sample S-7 as a reference. This value is presented so that the cholesterol synthesis inhibitory activities of the original plant samples for preparing the test materials can be compared with one another.

Theoretically, therefore, when doses of Test Sample S-7 and the positive control material (Mevalotin) which show an equivalent serum cholesterol lowering action are found from the results of a clinical test of administration of Test Sample S-7 and the positive control material to a hypercholesterolemia group, a suitable dose of each plant sample can be calculated based on the corrected titer.

In Test Example 1, the cholesterol synthesis inhibitory activity was found only in the water soluble fraction of each test sample. The ethanol fraction, acetone fraction and ethyl acetate fraction of each test sample were also tested for cholesterol synthesis inhibitory activity, but were found to have no such activity.

### Test Example 2 Human clinical test

Among the test samples found to have a hepatocyte cholesterol synthesis inhibitory activity in Test Example 1, Plant Samples T-1, T-5 and T-7 and Test Sample S-7 were used in a human clinical test on groups of hypercholesterolemia subjects, to determine the presence or absence of a clinical effect, and the correlation between the clinical effect and the cholesterol synthesis inhibitory activity.

### (1) Content of clinical test

(1-1) Eighty hypercholesterolemia subjects were divided into 4 groups. Each subject was given two cans daily of the test samples (canned drinks) shown in Table 4 for 4 weeks, to evaluate the improving effect on blood indices such as serum cholesterol level.
(1-2) Blood was taken before and after the 4-week test period (Week 0 and Week 4).
(1-3) During the test period, the subjects were allowed to continue their usual diet and activity, without dietary restrictions or programmed exercises.

### (2) Clinical test subjects

The clinical test was performed on hypercholesterolemia patients meeting the following criteria.

### (2-1) Selection criteria

i) Patients with a serum total cholesterol level of 240 (mg/dl) or higher
ii) Patients who understood the purpose and content of the test and consented to serve as subjects

### (2-2) Exclusion criteria

i) Patients with a complication of a hepatic disease, diabetes or like disease
ii) Patients given a therapeutic agent under treatment of hypercholesterolemia
iii) Patients regarded as unsuitable by the doctor for any other reasons

### (3) Test sample

Vegetable juices squeezed from Plant Samples T-1, T-5 and T-7 shown in Table 1 were filtered, and the filtrates were packed into cans (160 g per can), giving test samples. Further, another test sample was prepared by packing, per can, 160 g of a solution of 9.92 g of Test Sample S-7 (corresponding to 160 g of Plant Sample T-7) in purified water.

**Table 4**

| Test sample for Text Example 2 | | | | |
|---|---|---|---|---|
| Test sample | Content and its weight (g) | | Weight of final product (g) | Daily dose |
| 0-1 | T-1 | 60 | 160 | 2 cans (320 g) |
| 0-2 | T-2 | 60 | 160 | 2 cans (320 g) |
| 0-3 | T-7 | 60 | 160 | 2 cans (320 g) |
| 0-4 | S-7 | 0.92 | 160 | 2 cans (320 g) |

### (4) Test result

Table 5 shows the change and rate of reduction before and after the clinical test in total cholesterol level in the four test groups. Table 6 shows the change and rate of reduction in LDL cholesterol level.

**Table 5**

| Subject group | Change in total cholesterol level | | |
|---|---|---|---|
| | Week 0 (mg/dl) | Week 4 (mg/dl) | Rate of change (%) {Amount of change (mg/dl)} |
| Group 0-1 | 253.4±26.9 | 226.5±23.3** | ▲10.6 (26.9) |
| Group 0-2 | 252.6±23.1 | 243.8±21.5* | A 3.5 (8.8) |
| Group 0-3 | 252.4±27.9 | 223.3±24.6** | ▲11.5 (29.1) |
| Group 0-4 | 253.7±26.3 | 223.5±22.3** | ▲11.9 (30.2) |

| | | | |
|---|---|---|---|
| In the table, * indicates P<0.05, and ** indicates P<0.01. | | | |

**Table 6**

| Subject group | Change in LDL cholesterol level | | |
|---|---|---|---|
| | Week 0 (mg/dl) | Week 4 (mg/dl) | Rate of change (%) {Amount of change (mg/dl)} |
| Group 0-1 | 171.4±23.0 | 147.5±21.5** | ▲13.9 (23.9) |
| Group 0-2 | 168.2±26.5 | 160.5±23.3 | ▲4.6 (7.7) |
| Group 0-3 | 170.7±23.3 | 144.6±21.7** | ▲15.3 (26.1) |
| Group 0-4 | 171.0±24.8 | 143.3±21.1** | ▲16.2 (27.7) |

| | | | |
|---|---|---|---|
| In the table, ** indicates P<0.01. | | | |

Table 5 reveals that intake of the food of the present invention lowered the total cholesterol level in all of Groups 0-1 to 0-4.

In particular, the total cholesterol level in Group 0-4 was lowered by 11.9% on average, by 4-week intake. As compared with the rate of reduction achieved by different doses of Mevalotin (the positive control material used in Test Example 1), the rate of reduction in Group 0-4 was substantially equivalent to the rate of reduction achieved by 4-week administration of Mevalotin in a daily dose of 5 mg (11.1%). The prescribed daily dose of Mevalotin is 10 mg, which can lower the total cholesterol level by 18 to 20%.

The amount of change in cholesterol level shown in Tables 5 and 6 reveals that the main factor of the reduction in total cholesterol level is the reduction of LDL cholesterol.

Comparing the corrected titers shown in Table 3 and the results shown in Table 5 the rate of reduction in Group 0-3 (T-7) was ▲11.5%, which is nearly equal to the rate of reduction in Group 0-4 (S-7) of ▲11.9%. This substantially agrees with the fact that, in the case of Test Sample S-7, the ratio of the corrected titer to the original titer is 1:1.

The rate of reduction in Group O-1 (T-1) was ▲10.6% while the rate of reduction in Group 0-4 (S-7) was ▲11.9%. The ratio of the two values (▲10.6% : ▲11.9%) is 0.89:1, which is nearly equal to the corrected titer ratio of Test Sample S-1 to Test Sample S-7 (0.90:1). Further, the ratio of the rate of reduction in Group 0-5 (T-5) (▲3.5%) to the rate of reduction in Group 0-4 (S-7) (▲11.9%) is 0.29:1, which is nearly equal to the corrected titer ratio of Test Sample T-5 to Test Sample S-7 (0.34:1).

As to safety indices, no change was found in liver-related indices such as GOT, GPT, ALP, γ-GTP and LDH-5, or in cardiac muscle- or skeletal muscle-related indices such as CPK, LDH-1, LDH-2, LDH-3, LDH-4, creatine and creatinine, or in kidney-related indices such as BUN, creatinine and uric acid.

As to nutrition sufficiency indices, no change was found in serum total protein, albumin, immunoglobulin, choline esterase, serum amyrase or like indices, or in erythrocyte counts, hemoglobin level, platelet counts, hematocrit, leukocyte counts, leukocyte fractions (such as basophilic leukocytes, acidophilic leukocytes, lymphocytes and monocytes) or like indices.

As is apparent from the above, the human serum cholesterol lowering action of the food of the present invention is exhibited chiefly by the mechanism of hepatocyte cholesterol synthesis inhibitory activity. Namely, the food of the invention selectively reduces the low-density lipoprotein cholesterol (LDL-Chol) to thereby lower the serum total cholesterol, hence effective for improving lipid metabolism.

The serum cholesterol lowering action of the food of the invention was low as compared with Mevalotin, a medicine, but the food of the invention was completely free from side effects on the liver or cardiac muscle.

Therefore, the food of the invention is sufficiently significant for the purposes of keeping the good health of persons with slightly higher serum cholesterol level than normal level, since, according to the invention, human serum cholesterol can be lowered by taking a mixture of commonly eaten vegetables, which are free from the risk of unknown side effects usually entailed by novel medicines.

### Comparative Example 1

The following are the results of a test wherein rats were given a mixed vegetable juice containing suitable amounts of broccoli, cabbage, Japanese radish leaves, celery, lettuce, spinach, *komatsuna,* parsley and apple.

Ten SD-SPF rats (male, 4-week old) were divided into 2 groups. Group A (control group) was fed only a standard diet ad libitum, while Group B (test group) was fed a standard diet and the mixed vegetable juice ad libitum.

Generally, excessively high-fat diets such as fat-loaded diets are not employed during prophylactic or therapeutic treatment for lowering the cholesterol level, even when the treatment do not involve dietary restrictions. In the test examples of this invention, therefore, the rats were fed a standard diet, not a fat-loaded diet, to evaluate the serum cholesterol lowering action of the mixed vegetable juice.

Table 7 shows the total cholesterol level in each group before and 4 weeks after the start of the test.

**Table 7**

| Subject group | Change in total cholesterol level | | |
|---|---|---|---|
| | Week 0 (mg/dl) | Week 4 (mg/dl) | Rate of change (%) [Amount of change (mg/dl)] |
| Group A | 111.8 | 74.2 | ▲ 33.6 (37.6) |
| Group B | 114.6 | 92.2 | ▲ 19.5 (22.4) |

The total cholesterol level lowered not only in Group B given the mixed vegetable juice in addition to the standard diet, but also in Group A given only the standard diet. Rather, the amount and rate of change in Group A, which was fed the standard diet alone, were greater than those in Group B.

The above results demonstrate that the mixed vegetable juice containing broccoli, cabbage, Japanese radish leaves and other vegetables does not have an action of lowering the total cholesterol of rats.

The following are formulation examples of foods and medicines according to the present invention.

### Formulation Example 1 Tablets

| | |
|---|---|
| Lactose | 80.0% |
| Gelatin | 10.0% |
| Freeze-dried broccoli | 1.0% |
| Freeze-dried cabbage | 1.0% |
| Fruit flavor | 0.5% |
| Water | 7.5% |

### Formulation Example 2 Beverage

| | |
|---|---|
| Apple juice | 20.0 % |
| Broccoli puree | 20.0 % |
| Cabbage juice | 10.0 % |
| Concentrated lemon juice | 0.5 % |
| Pigment | 0.05% |
| Flavor | 0.5 % |
| Water | 48.95% |

## Claims

1. A food product comprising broccoli and cabbage for use in lowering higher than normal human serum cholesterol.

2. The food product for use according to claim 1, wherein the food further contains Japanese radish leaves or Chinese cabbage.

3. The food product for use according to claim 1, wherein the food further contains at least one member selected from the group consisting of cauliflower, kale, shepherd's purse, *suzushiro* (*Arabis flagellosa* Miq.), *hatazao* (*Arabis glabra), tagarashi* (*Cardamine flexuosa*), woad, *oobatanetsukebana* (*Cardamine scutata), yamagarashi* (*Barbarea vulgaris* R. Br. var. *stricta* Regel), watercress, *takana (Brassica juncea* Czern. et Coss. var. *integrifolia* Skinsk.), *karashina* (*Brassica juncea* Czern. et Coss.), Japanese horseradish, *yuriwasabi (Wasabia tenius), hinona* (*Brassica campestris* var. *akana*), *sugukina* (*Brassica campestris* var. *neosuguki*), turnip, rape, spinach, *komatsuna (Brassica rapa* L.), celery, parsley, lettuce, apple, Chinese cabbage, brussels sprouts, Japanese radish and Japanese radish leaves.

4. The food product for use according to claim 1, wherein the food further contains at least one member selected from the group consisting of cauliflower, kale, shepherd's purse, *suzushiro (Arabis flagellosa* Miq.), *hatazao (Arabis glabra), tagarashi (Cardamine flexuosa),* woad, *oobatanetsukebana (Cardamine scutata), yamagarashi (Barbarea vulgaris* R. Br. var. *stricta* Regel), watercress, *takana (Brassica juncea* Czern. et Coss. var. *integrifolia* Skinsk.), *karashina* (*Brassica juncea* Czern. et Coss.), Japanese horseradish, *yuriwasabi* (*Wasabia tenius), hinona* (*Brassica campestris* var. *akana*), *sugukina (Brassica campestris* var. *neosuguki*), turnip, rape, spinach, *komatsuna (Brassica rapa* L.), celery, parsley, lettuce and apple.

5. The food product for use according to claim 1, wherein the food further contains at least one member selected from the group consisting of shepherd's purse, *suzushiro* (*Arabis flagellosa* Miq.), *hatazao* (*Arabis glabra), tagarashi* (*Cardamine flexuosa*), woad, *oobatanetsukebana* (*Cardamine scutata*), *yamagarashi* (*Barbarea vulgaris* R. Br. var. *stricta* Regel), watercress, *takana* (*Brassica juncea* Czern. et Coss. var. *integrifolia* Skinsk.), *karashina* (*Brassica juncea* Czern. et Coss.), Japanese horseradish, *yuriwasabi* (*Wasabia tenius*), Chinese cabbage, *hinona* (*Brassica campestris* var. *akana*), *sugukina* (*Brassica campestris* var. *neosuguki*), turnip, rape, brussels sprouts, Japanese radish and Japanese radish leaves.

6. The food product for use according to any of claims 1-5, wherein the food further contains at least one member selected from the group consisting of brussel sprouts, Japanese radish and Japanese radish leaves, and at least one of shepherd's purse, *suzushiro, hatazao, tagarashi,* woad, *oobatanetsukebana, yamagarashi,* watercress, *takana, karashina,* Japanese horseradish, *yuriwasabi,* Chinese cabbage, *hinona, sugukina,* turnip and rape.

7. The food product for use according to any of claims 3-6, wherein the at least one member is a fruit or vegetable and in a raw state, or is thermally sterilised.

8. The food product for use according to claim 7, wherein the food is a powder.

9. The food product for use according to any of claims 1-3 and 7-8, wherein the food is a vegetable or fruit juice, or a concentrate of a vegetable or fruit juice.

## Patentansprüche

1. Lebensmittelerzeugnis, umfassend Brokkoli und Kohl, zur Verwendung um höher als normales Humanserumcholesterin zu senken.

2. Lebensmittelerzeugnis zur Verwendung nach Anspruch 1, wobei das Lebensmittel weiter japanische Rettichblätter oder Chinakohl enthält.

3. Lebensmittelerzeugnis zur Verwendung nach Anspruch 1, wobei das Lebensmittel weiter mindestens ein Mitglied, ausgewählt aus der Gruppe, bestehend aus Blumenkohl, Grünkohl, gewöhnlichem Hirtentäschel, *suzushiro (Arabis flagellosa Miq.), hatazao* (*Arabis glabra*), *tagarashi (Cardamine flexuosa),* Waid, *oobatanetsukebana* (*Cardamine scutata*), *yamagarashi* (*Barbarea vulgaris* R. Br. Var. *stricta* Regel), Wasserkresse, *takana* (*Brassica juncea* Czern. et Coss. var. *integrifolia* Skinsk.), *karashina (Brassica juncea* Czern. et Coss.), japanischem Meerrettich, *yuriwasabi (Wasabia tenius), hinona* (*Brassica campestris* var. *akana*), *sugukina* (*Brassica campestris* var. *neosuguki*), Steckrübe, Raps, Spinat, *komatsuna* (*Brassica rapa* L.), Sellerie, Petersilie, Salat, Apfel, Chinakohl, Rosenkohl, japanischem Rettich und japanischen Rettichblättern, enthält.

4. Lebensmittelerzeugnis zur Verwendung nach Anspruch 1, wobei das Lebensmittel weiter mindestens ein Mitglied, ausgewählt aus der Gruppe, bestehend aus Blumenkohl, Grünkohl, gewöhnlichem Hirtentäschel, *suzushiro* (*Arabis flagellosa Miq.*), *hatazao* (*Arabis glabra*), *tagarashi* (*Cardamine flexuosa),* Waid, *oobatanetsukebana* (*Cardamine scutata*), *yamagarashi* (*Barbarea vulgaris* R. Br. Var. *stricta* Regel), Wasserkresse, *takana (Brassica juncea* Czern. et Coss. var. *integrifolia* Skinsk.), *karashina* (*Brassica juncea* Czern. et Coss.), japanischem Meerrettich, *yuriwasabi* (*Wasabia tenius*), *hinona (Brassica campestris* var. *akana*), *sugukina* (B*rassica campestris* var. *neosuguki),* Steckrübe, Raps, Spinat, *komatsuna* (*Brassica rapa* L.), Sellerie, Petersilie, Salat und Apfel, enthält.

5. Lebensmittelerzeugnis zur Verwendung nach Anspruch 1, wobei das Lebensmittel weiter mindestens ein Mitglied, ausgewählt aus der Gruppe, bestehend aus gewöhnlichem Hirtentäschel, *suzushiro* (*Arabis flagellosa Miq*.)*, hatazao* (*Arabis glabra*), *tagarashi* (*Cardamine flexuosa*), Waid, *oobatanetsukebana* (*Cardamine scutata*), *yamagarashi* (*Barbarea vulgaris* R. Br. Var. *stricta* Regel), Wasserkresse, *takana* (*Brassica juncea* Czern. et Coss. var. *integrifolia* Skinsk.), *karashina (Brassica juncea* Czern. et Coss.), japanischem Meerrettich, *yuriwasabi* (*Wasabia tenius*), Chinakohl, *hinona* (*Brassica campestris* var. *akana*), *sugukina (Brassica campestris* var. *neosuguki),* Steckrübe, Raps, Rosenkohl, japanischem Rettich und japanischen Rettichblättern, enthält.

6. Lebensmittelerzeugnis zur Verwendung nach einem der Ansprüche 1 bis 5, wobei das Lebensmittel weiter mindestens ein Mitglied, ausgewählt aus der Gruppe, bestehend aus Rosenkohl, japanischem Rettich und japanischen Rettichblättern und mindestens eines von gewöhnlichem Hirtentäschel, *suzushiro, hatazao, tagarashi,* Waid, *oobatanetsukebana, yamagarashi,* Wasserkresse, *takana, karashina,* japanischem Meerrettich, *yuriwasabi,* Chinakohl, *hinona, sugukina,* Steckrübe und Raps enthält.

7. Lebensmittelerzeugnis zur Verwendung nach einem der Ansprüche 3 bis 6, wobei das mindestens eine Mitglied eine Frucht oder Gemüse ist und in einem rohen Zustand oder thermisch sterilisiert ist.

8. Lebensmittelerzeugnis zur Verwendung nach Anspruch 7, wobei das Lebensmittel ein Pulver ist.

9. Lebensmittelerzeugnis zur Verwendung nach einem der Ansprüche 1 bis 3 und 7 bis 8, wobei das Lebensmittel ein Gemüse- oder Fruchtsaft oder ein Konzentrat eines Gemüse- oder Fruchtsafts ist.

## Revendications

1. Produit alimentaire comprenant du brocoli et du chou pour une utilisation dans la réduction du taux de cholestérol sérique humain plus élevé que la normale.

2. Produit alimentaire pour une utilisation selon la revendication 1, dans lequel l'aliment contient en outre des feuilles de daïkon ou du chou chinois.

3. Produit alimentaire pour une utilisation selon la revendication 1, dans lequel l'aliment contient en outre au moins un élément choisi dans le groupe constitué par le chou-fleur, le chou frisé, la capselle, le *suzushiro* (*Arabis flagellosa* Miq.), *l'hatazao* (*Arabis glabra*), le *togarashi* (*Cardamine flexuosa*), le pastel, *l'oobatanetsukebana* (*Cardamine scutata*), le *yamagarashi* (*Barbarea vulgaris* R. Br. var. *stricta* Regel), le cresson d'eau, le *takana (Brassica juncea* Czern. et Coss. var. *integrifolia* Skinsk.), le *karashina (Brassica juncea* Czern. et Coss.), le wasabi, le *yuriwasabi* (*Wasabia tenius*), *l'hinona (Brassica campestris* var. *akana),* le *sugukina (Brassica campestris* var. *neosuguki),* le navet, la navette, l'épinard, le *komatsuna (Brassica rapa* L.), le céleri, le persil, la laitue, la pomme, le chou chinois, le chou de Bruxelles, le daïkon et les feuilles de daïkon.

4. Produit alimentaire pour une utilisation selon la revendication 1, dans lequel l'aliment contient en outre au moins un élément choisi dans le groupe constitué par le chou-fleur, le chou frisé, la capselle, le *suzushiro* (*Arabis flagellosa* Miq.), *l'hatazao* (*Arabis glabra*), le *togarashi* (*Cardamine flexuosa*), le pastel, *l'oobatanetsukebana* (*Cardamine scutata*), le *yamagarashi (Barbarea vulgaris* R. Br. var. *stricta* Regel), le cresson d'eau, le *takana (Brassica juncea* Czern. et Coss. var. *integrifolia* Skinsk.), le *karashina* (*Brassica juncea* Czern. et Coss.), le wasabi, le *yuriwasabi (Wasabia tenius), l'hinona (Brassica campestris* var. *akana*), le *sugukina* (*Brassica campestris* var. *neosuguki*), le navet, la navette, l'épinard, le *komatsuna* (*Brassica rapa* L.), le céleri, le persil, la laitue et la pomme.

5. Produit alimentaire pour une utilisation selon la revendication 1, dans lequel l'aliment contient en outre au moins un élément choisi dans le groupe constitué par la capselle, le *suzushiro* (*Arabis flagellosa* Miq.), *l'hatazao* (*Arabis glabra*), le *togarashi* (*Cardamine flexuosa*), le pastel, *l'oobatanetsukebana* (*Cardamine scutata*), le *yamagarashi* (*Barbarea vulgaris* R. Br. var. *stricta* Regel), le cresson d'eau, le *takana (Brassica juncea* Czern. et Coss. var. *integrifolia* Skinsk.), le *karashina (Brassica juncea* Czern. et Coss.), le wasabi, le *yuriwasabi (Wasabia tenius), l'hinona (Brassica campestris* var. *akana),* le *sugukina (Brassica campestris* var. *neosuguki),* le navet, la navette, le chou de Bruxelles, le daïkon et les feuilles de daïkon.

6. Produit alimentaire pour une utilisation selon l'une quelconque des revendications 1 à 5, dans lequel l'aliment contient en outre au moins un élément choisi dans le groupe constitué par le chou de Bruxelles, le daïkon et les feuilles de daïkon, et au moins l'un de la capselle, du *suzushiro,* de *l'hatazao,* du *tagarashi,* du pastel, de *l'oobatanetsukebana,* du *yamagarashi,* du cresson d'eau, du *takana,* du *karashina,* du wasabi, du *yuriwasabi,* du chou chinois, de *l'hinona,* du *sugukina,* du navet et de la navette.

7. Produit alimentaire pour une utilisation selon l'une quelconque des revendications 3 à 6, dans lequel l'au moins un élément est un fruit ou un légume et est à l'état cru, ou est thermiquement stérilisé.

8. Produit alimentaire pour une utilisation selon la revendication 7, dans lequel l'aliment est une poudre.

9. Produit alimentaire pour une utilisation selon l'une quelconque des revendications 1 à 3 et 7 à 8, dans lequel l'aliment est un jus de légume ou de fruit, ou un concentré d'un jus de légume ou de fruit.
